# EUROPEAN PATENT APPLICATION

(11) **EP 3 376 415 A1**
(43) Date of publication of application: **19.09.2018**
(21) Application number: 17160516.5
(22) Date of filing: 13.03.2017
(51) Int. Cl.: G06F 19/00, G09B 23/32, G06T 15/00, G06T 17/00

(54) **A SYSTEM FOR SIMULATING VISUAL ACCOMODATION**

(71) Applicant: KEJAKO SA, 1228 Plan-les-Ouates (CH)
(72) Inventor: BOS, Gilles, 1228 Plan-les-Ouates (CH); MAURER, Aurélien, 1228 Plan-les-Ouates (CH); ENFRUN, David, 1228 Plan-les-Ouates (CH); MAITRE, Pierre-Yves, 1228 Plan-les-Ouates (CH); DELAGE, Gabriel, 1228 Plan-les-Ouates (CH); ZUBER, Charles-Olivier, 1228 Plan-les-Ouates (CH); CARTA, Mario, 1228 Plan-les-Ouates (CH)
(74) Representative: Petit, Maxime

(57) **Abstract**

A system (100) for simulating visual accommodation comprising one or more processors and one or more computer-readable storage media encoded with instructions that, when executed by at least one of the processors, cause the computer system to enable a geometry module (101), a physics module (102), a simulation engine (103), an outputs providing module (104), an update module (105) and a interface module (106).

## Description

### FIELD OF THE INVENTION

The present invention pertains to the field of systems for simulating the functioning of a human eye. The invention concerns in particular a computer system for simulating a process of visual accommodation.

### BACKGROUND OF THE INVENTION

The mechanism of visual accommodation, the process by which the vertebrate eye changes optical power to maintain a clear image or focus on an object as its distance varies, has been studied since the early 17th century when, according to Kepler, the accommodative process was activated by a deformation of the Retina. Then, around the same period, Descartes suggested in his work a possible axial displacement of the lens for focusing. This mechanism was not the one of human and mammal species but does really exist in most species of fish. Since then, a lot of theories have been made to explain the ability to see at different distance. Today's the most commonly accepted is based on the Helmholtz's one. In the 19th century Von Helmholtz proposed a description of the accommodation mechanism based on the deformation of the crystalline lens, flattened and equatorially stretched for far vision, and round for near vision. In the 20th century, other theories of the accommodation have been proposed, for instance Coleman's theory about the influence of gradient of intra ocular pressure on the lens shape, Schachar's theory on the lens mechanical stretching leading to a paradoxical central increase of curvature due to non-intuitive selective tensions on zonula or Goldberg's theory on a zonular reciprocal action, which is an improvement of the Helmholtz's theory. Today's scientific community mostly acknowledges the Helmholtz's theory as "globally" the valid one, but most of the descriptions of the process are not clear and some points are still discussed.

In relation to visual accommodation, presbyopia is the consequence of natural age related gradual loss of the accommodative amplitude of the human eye. Generally, around the age of forty-five, people start struggling to see close object and their vision tends to be limited to far vision only. Over a billion people around the world are at different degree dealing with this aging process. Research showed that presbyopia is, on one hand, linked to stiffness and loss of deformation amplitude and, on the other hand, to the so-called "lens paradox". With age, the crystalline lens gets indeed stiffer, which means that, for same effort of equatorial stretching (such as the effort that happen to achieve far vision), the deformation will be lower. The difference between the less and the most accommodated shape shortens, and the « far vision shape » is then rounder in the old eye than in the young one. Although this should lead to a close-range vision, the decrease of the refractive index maintains the ability to see far object. This refractive offset, however, decreases the ability to see close objects.

As presbyopia relates to some deterioration in the visual accommodation process, computer systems have been designed to allow the provision of simulations of the functioning of an eye, more precisely simulations of the process of visual accommodation. However, the computer systems for simulating visual accommodation available at present are either limited, because they rely on too few physiological entities and physics, or inefficient, because of the large amount of computational resources they need to operate or the computational time they require for providing results. These downsides drastically decrease the interest for making use of such systems in a therapeutic environment or for purposes of research and development of therapeutic solutions.

### SUMMARY OF THE INVENTION

The present invention intends to remedy at least one of these disadvantages.

One goal of the invention is to provide a computer system for simulating visual accommodation, i.e. a computer system able to simulate a process of an eye switching between far vision and near vision and able to provide graphical and/or numerical outputs in this regard, that mimics more accurately the physiological reality of the visual accommodation process as it occurs in nature.

The system of the invention may also address the need for medical actors to use the system in relation to in-vivo cases and real therapeutic constraints. Thus, the system for simulating visual accommodation of the invention may facilitate the submission and the processing of modifications in relation to the geometry and/or to the material properties of the physiological entities that form an eye.

Moreover, while it must be possible to use the system standalone, for instance for research and developments purposes, it must also be possible to make use of the system in a computerized therapeutic environment, for instance in relation to a medical imaging device or to a medical instrument used for the treatment of visual disorders. Thus, the computer system for simulating visual accommodation of the invention must include functionalities to enable an interface through which it may be connected via a communication link to a remote computer, with which data may be exchanged. Moreover, for the sake of efficiency in such situations where the system is part of a distributed environment, it must implement mechanisms for automatic data retrieval and to ensure that simulation outputs that are provided are automatically and continuously updated in accordance with data retrieved and/or submitted.

At least one of these goals is achieved by a computer system for simulating visual accommodation as defined in claim 1.

The computer system for simulating visual accommodation of the present invention more accurately mimics the physiological reality because simulation outputs provided by the system rely on a three-dimensional geometry of an eye that includes and distinctively addresses all important physiological entities of the eye, which are at least the crystalline lens, the zonula, the ciliary muscle, the sclera and the cornea. More accurate outputs provided by the system according to the invention result also from the specific way of modeling physiological entities of an eye, from the ability to specify numerous parameters with respect to each physiological entity of an eye, which parameters may define mechanical or optical properties in relation to those entities, and from the variety of results (forces, displacements, optical parameters for specific areas or volumes) that are provided. Greater accuracy and greater efficiency are also achieved because of the system configuration to establish a simulation environment which combines geometrical inputs and parameters characterizing material's properties in a specific manner, with the ability to submit and immediately consider changes and modifications applied to the simulation environment. The computer system of the present invention further provides improvements in terms of user interaction because it is configured to automatically determines whether data must be retrieved from remote data sources. All these aspects allow the system of the present invention to be more efficient when used in a therapeutic environment, especially for surgical planning procedures or diagnosis of residual accommodation capability.

Further possible features of the computer system according to the invention are defined by claims 2-15.

### BRIEF DESCRIPTION OF THE FIGURES

The present invention will be better understood by reading the following description in relation to the drawings which show:
- figure 1, a schematic view of the major components of the computer system according to one embodiment of the invention;
- figure 2, a schematic view of a window displayed in the graphical user interface of the system according to one embodiment of the invention;
- figure 3, a schematic view of a process implemented by the system according to one embodiment of the invention;
- figure 4, a schematic view of another window displayed in the graphical user interface of the system according to one embodiment of the invention;
- figure 5, a schematic view of another process implemented by the system according to one embodiment of the invention; and
- figure 6, a schematic view illustrating how a system according to one embodiment of the invention may be applied in therapeutic environment.

### DETAILED DESCRIPTION OF THE INVENTION

The computer system for simulating visual accommodation according to the invention (hereinafter referred as "the system") comprises at least one processor and at least one computer-readable storage media. In a first embodiment of the invention, the system includes a microcomputer whereas, in a second embodiment, it includes a professional workstation, a server, a mainframe, a supercomputer or a combination of such systems. The computer-readable storage media of the system, for instance a memory, is encoded with instructions that, when executed by the processor, enable a simulation system for simulating visual accommodation, whose parameters, models, computations, results and advantages are described below.

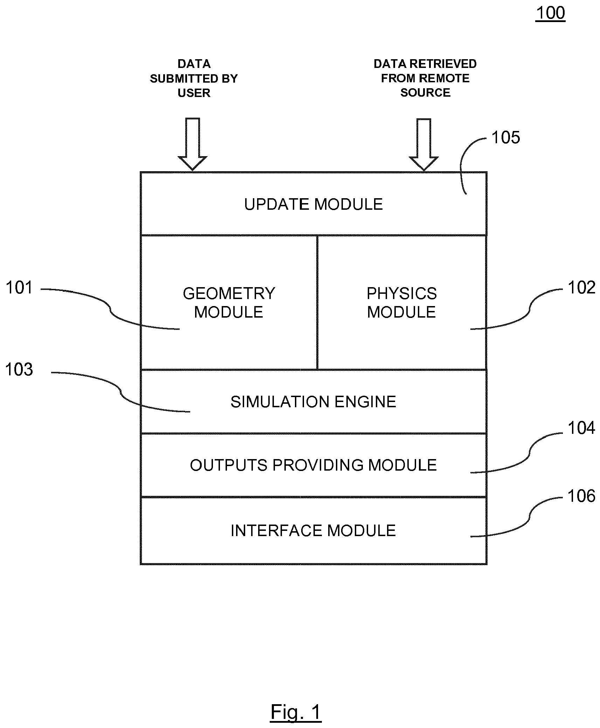

As shown on figure 1, the system 100 preferably comprises five components, that are a geometry module 101, a physics module 102, a simulation engine 103, an output providing module 104, an update module 105 and an interface module 106.

The geometry module 101 allows to set a three-dimensional geometrical model of an eye. The three-dimensional geometrical model delineates volumetric boundaries of several physiological entities of an eye. The physiological entities whose volumetric boundaries are thus distinctively defined by the geometrical model are, preferably, the crystalline lens, the zonula, the ciliary muscle, the sclera, the cornea, the vitreous body and the aqueous humor. Accordingly, the geometrical model defined by the geometry module 101 results from an assemblage of several distinct geometries, each one relating to a specific physiological entity of an eye.

According to the invention, all physiological entities involved in the process of visual accommodation are defined by the geometrical model specified by the geometry module 101 of the system 100. As such, the geometry module 101 allows to faithfully transpose the geometry of a real eye and, therefore, it contributes to greater accuracy of the simulation results of visual accommodation which are provided by the system 100.

The crystalline lens as defined by the geometrical model is divided in four subparts, which are the nucleus, the lens cortex, the lens capsule and the lens epithelium. From a geometrical point of view, each subpart is defined as a solid generated by cross sectional continuous curves, forming a bi-convex aspheric and not necessarily regular domain. Each curve is the representation of a 5^{th} order polynomial I, where I is the considered plan of the cross section. The crystalline lens is thus defined as a set of four of such volumes nested together. The geometrical model further defines an offset of distance between each subpart along the crystalline lens polar axis

With respect to the sclera and the cornea, the geometrical model defines the sclera's shape and the cornea's shape as a volume generated by an ensemble of 5^{th} order polynomial curves.

With respect to the zonula, since the zonular fibers form a complex web sustaining the lens, connecting it with the ciliary muscle, and linking the ciliary muscle to the sclera, the geometrical model defines three zonula groups (posterior, equatorial, anterior). Each group is made of two zonula rings delimiting the insertion thickness. The geometrical model further defines several intersections where each of these groups intersects with the crystalline lens and/or the ciliary muscle.

With respect to the ciliary muscle, which is a structure of multiple fibers orientation, the geometrical model defines its specific shape as bounded by a skin, which is defined through a generated central part of revolution from which are derived multiple discrete segments of extrusion.

The geometrical model further defines at least one area where the ciliary muscle is fixed to the sclera, points or areas at which it interacts with the zonula and further geometrical aspects designed to allow the ciliary muscle to slide on the choroid during the accommodation process.

The geometry module 101 interacts with the interface module 106 to generate a window of the graphical user interface provided by the interface module 106, in which a three-dimensional representation of the geometrical model is displayed.

The graphical user interface generated by the interface module 106 provides functionalities enabling data submission, including numerical and graphical inputs. Accordingly, the geometrical model managed by the geometry module 101 may be modified by means of graphical inputs performed directly on the three-dimensional representation displayed in the graphical user interface, thereby allowing a sort of "WYSIWYG - what you see is what you get" type of interaction. This feature is particularly advantageous in situations where, for instance, the geometrical model must be aligned to in-vivo measurements of real eyes' geometries. In that sense, the system 100 provides an improvement in terms of man-to-machine interaction, thereby enhancing its efficiency when used, for instance, in research & development or therapeutic environments.

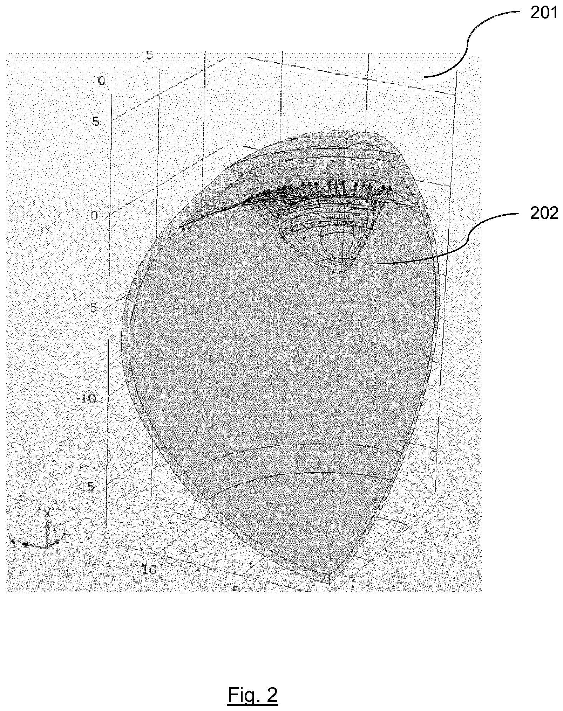

Figure 2 schematically depicts at least a part of a window 201 of the graphical user interface provided by the interface module 106, in which a partial three-dimensional representation 202 of the geometrical model is displayed.

The role of the physics module 102 is to define a physics-related environment in relation to the geometrical model managed by the geometry module 101. As such, it allows the simulation results provided by the system 100 to rely on a combination of geometry and physics, by means of a precise definition of mechanical and optical properties in relation to given eyes' geometries.

To this end, the physics module 102 allows setting of numerous physics-related parameters to characterize optical and mechanical properties of, preferably, all parts defined by the geometrical model.

Advantageously, the physics module 102 processes data to assign mechanical and/or optical properties to specific points, areas or volumes defined by the geometrical model. Thus, the physics module 102 provides fine tuning capabilities for setting the physics-related environment, thereby contributing to the provision by the system of more accurate simulation results.

With respect to the mechanical properties of the crystalline lens, the physics module 102 allows to set a physics-related parameter that characterizes its stiffness. Preferably, the physics module 102 allows to set distinct physics-related parameters, some or all relating to stiffness, with respect to specific areas of the nucleus, the lens cortex, the lens capsule or the lens epithelium. Advantageously, a physics-related parameter may be set only for specific points, areas or volumes of those subparts.

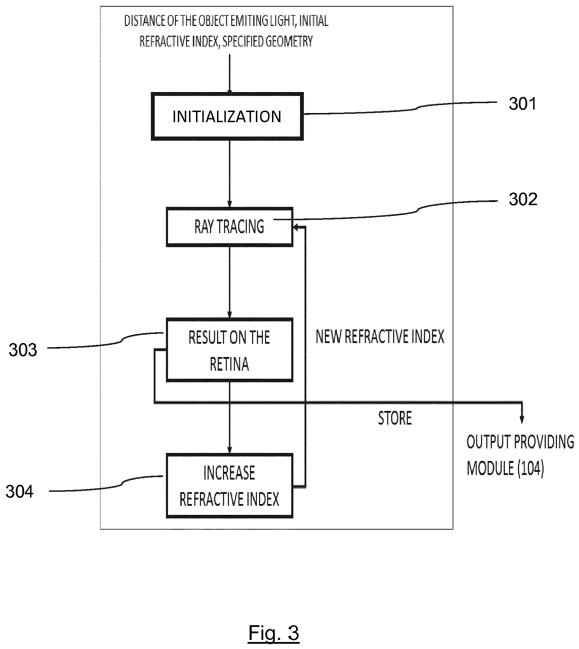

With respect to the optical properties of the crystalline lens, the physic module 102 allows a physics-related parameter of refractive index to be set. Preferably, setting of the refractive index parameter involves determination of the refractive index parameter based on a computation of the sum of cumulated light received by the retina, for different concentric areas characterizing the sharpness of focus, when considering, for a given geometry, a situation where light is emitted by a point located one meter away from the cornea. This computation that may be performed by the physics module 102 in order to determine the refractive index parameter is depicted by figure 3.

In a step 301, the physics module 102 retrieves the geometrical model defined by the geometry module 101, a value characterizing the distance of the object emitting light and assigns an initial value to the refractive index parameter. In a step 302, the physics module 102 generates a ray tracing and, in a step 303, the physics module 102 determines a corresponding result on the retina by computing the cumulated light received by the retina. In a step 304, the physics module 102 increases the refractive index parameter by a pre-defined offset and repeats the steps 302 and 303 until a defined stop value of refractive index. Once it has performed those steps for several different values of refractive index, the physics module 102 sets the refractive index parameter by selecting the value which produces the sharpest result on the retina.

With respect to the mechanical properties of the sclera, the physics module 102 allows to set a physics-related parameter to characterize its stiffness, preferably by means of a linear material model.

With respect to mechanical properties of the zonula, the physics module 102 allows to set a physics-related parameter that characterizes the stiffness of its anterior, posterior and equatorial parts. Preferably, the physics module 102 allows setting of a physics-related parameter that characterizes an elongation level of the posterior part of the zonula.

With respect to mechanical properties of the ciliary muscle, the physics module 102 allows to set a physics-related parameter that characterizes its core's elasticity and its skin's rigidity. Moreover, the physics module 102 allows to set a physics-related parameter that characterizes weaknesses at some specific locations of the muscle's skin, thereby allowing to characterize the behavior of the muscle's skin which, during accommodation, changes its shape when submitted to the traction of the posterior part of the crystalline lens. Preferably, the physics module 102 also allows setting of a physics-related parameter that characterizes contraction and/or elongation level with respect to the whole ciliary muscle and/or with respect to only a part of it. Accordingly, the visual accommodation process may be simulated by means of modifications applied to the physics-related parameters characterizing stiffness of at least some parts of the ciliary muscle.

With respect to mechanical properties of the aqueous humor, the physics module 102 allows setting of a physics-related parameter that characterizes its dynamic viscosity, density or elasticity. Moreover, the physics module 102 allows to set several physics-related parameters that characterize fluidic properties of anterior and posterior chambers, inflow from the ciliary body at a constant mass flow rate and an outflow rate.

With respect to optical properties of the aqueous humor, the physics module 102 allows a physics-related parameter that characterizes its refractive index to be set.

With respect to mechanical properties of the vitreous body, the physics module 102 allows a physics-related parameter that characterizes its elasticity and/or an index of refraction to be set. Preferably, viscoelastic properties of the vitreous body are characterized by the physics-related module 102 via a physics-related parameter which is based on two Kelvin-Voigt models in chain with a damper.

With respect to optical properties of the vitreous body, the physics module 102 allows a physics-related parameter that characterizes its refractive index to be set.

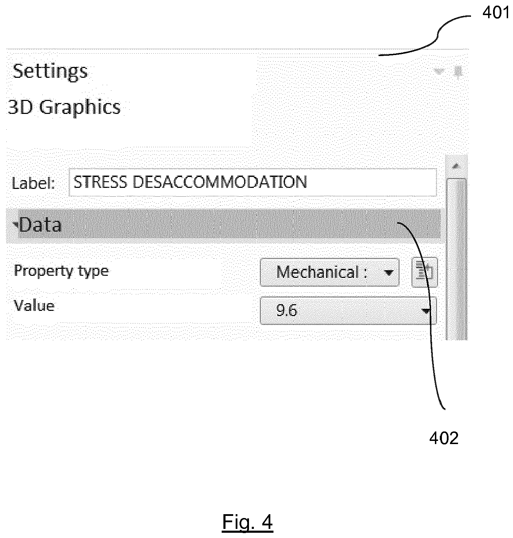

In terms of man-to-machine interaction, the physics module 102 interacts with the interface module 106 to generate, as shown by figure 4, a window 401 of the graphical user interface provided by interface module 106. The window 401 includes at least input means 402 that may be operated to adjust all the physics-related parameters defined by the physics module 102. Alternatively, or cumulatively, the window 401 includes input areas to facilitate numerical inputs, thereby allowing also a "WYSIWYG" type of man-to-machine interaction. As mentioned above, the graphical user interface provides functionalities for management of any type of man-to-machine interactions. This applies to the window 401, which may thus allow graphical inputs to be submitted by means of pointing devices and numerical inputs to be specified by means of dedicated input areas.

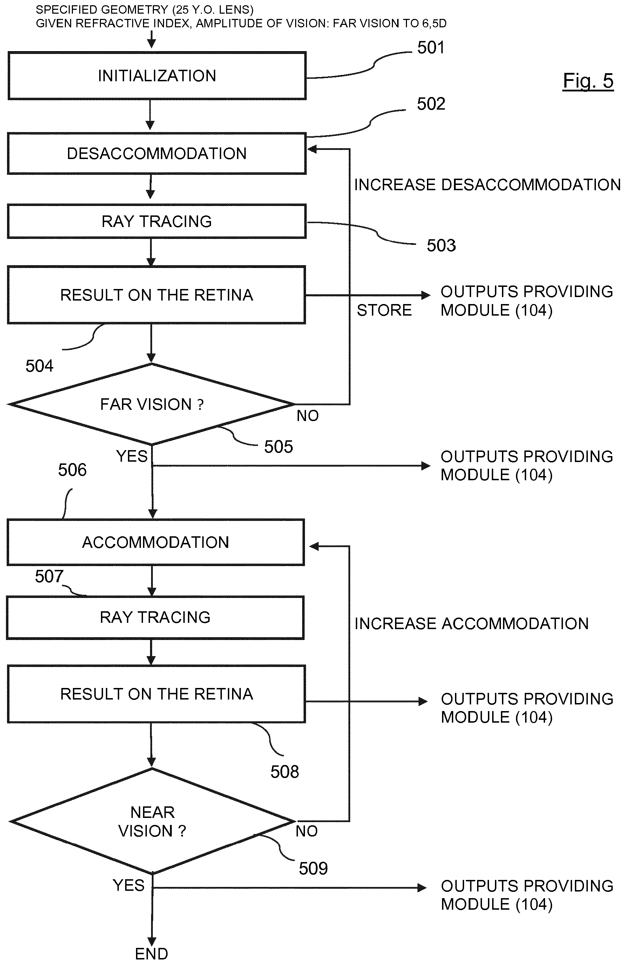

The role of the simulation engine 103 is to combine the geometrical model defined by the geometry module 101 with the physics-related environment defined by the physics module 102. As such, the simulation engine 103 generates a simulation model which, to produce graphical or numerical results with respect to a process a visual accommodation, duly considers geometrical and physics-related aspects. An example of a process of combining geometrical and physics-related aspects that may be performed by the simulation engine 103 is depicted by figure 5.

In a first step 501, the simulation engine 103 retrieves from the geometry module 101 a geometrical model characterizing a young emmetropic eye, preferably a twenty-five years old eye. In parallel, the simulation engine 103 retrieves from the physics module 102 the refractive index parameter of the crystalline lens corresponding to the given geometry.

In a step 502, the simulation engine 103 operates the geometrical model to characterize a tension on the posterior part of the zonula, which induces a deformation of the crystalline lens and, with respect to the given geometry of a twenty-five years old eye, the simulation engine 103 determines the force needed to achieve far vision. Accordingly, during this step 502, the simulation engine 103 sets an initial value of tension applied on a posterior part of the zonula to achieve far vision. In a step 503, the simulation engine 103 generates a ray tracing and, in a step 504, it determines a corresponding result on the retina by computing the cumulated light received by the retina. In a step 505, the simulation engine 103 determines if far vision is achieved or not. If it is not the case, in order to characterize greater tension applied on the posterior part of the zonula, the simulation engine 103 increases the value of tension applied on the posterior part of the zonula using a pre-defined offset and steps 502-505 are repeated until the simulation engine 103 determines in step 505 that far vision is achieved. When the simulation engine 103 determines that far vision is achieved, it transmits the value of tension applied on the posterior part of the zonula to achieve far vision to the outputs providing module 104.

Then, in a step 506, considering the far vision achieved, the previous determined value of tension applied on posterior part of the zonula to achieve far vision remain identical, and the simulation engine 103 applies a first initial value of contraction of the ciliary muscle. In a step 507, the simulation engine 103 generates another ray tracing for a given distance of ray-emission corresponding to a close object and, in a step 508, it determines the result on the retina. Then, in a step 509, the simulation engine 103 determines if near vision is achieved or not. If it is not the case, in order to characterize greater contraction value of the ciliary muscle, the simulation engine 103 increases the value of contraction applied on the ciliary muscle using a pre-defined offset and steps 506-509 are repeated until the simulation engine 103 determines in step 509 that near vision is achieved. When the simulation engine 103 determines that near vision is achieved, it transmits the value of contraction applied on the ciliary muscle to achieve near Vision to the outputs providing module 104.

By means of this process, the simulation engine 103 is thus able to determine the parameters of the tension and contraction characterizing an accommodation amplitude of a young eye. Moreover, considering the hypothesis that the efforts for visual accommodation remain similar for young and old people, the simulation engine 103 may for instance evaluate the accommodation amplitude of an old eye by applying the previously determined parameters of tension and contraction to the geometrical model and the physics related environment of an older eye.

The role of the output providing module 104 is to retrieve results data that are generated by the simulation engine 103 and to interact with the interface module 106 to present these results as numerical and graphical outputs.

The simulation results generated by the simulation engine 103 and retrieved by the outputs providing module 104 include, preferably with respect to all physiological entities defined by the geometrical model, data that relates to forces, mechanical stresses, deformations or displacements. With respect to optical domains, the results include data that relate to optical changes and, in relation to visual accommodation, the results retrieved by the outputs providing module 104 include at least data defining a value of accommodation amplitude.

The outputs providing module 104 retrieves data from the simulation engine 103 and processes the data to feed the interface module 106. In this respect, the interface module 106 may present the results via one or more windows. Preferably, results are presented in a graphical or numerical form, the choice of format being made in accordance with a set of pre-defined formats which specifies, for each result that may be provided by the simulation engine 103, an appropriate format to be used.

A role of the update module 105 is to monitor user inputs and, if necessary, to update the geometrical model or to the physics-related environment accordingly. In this respect, the update module 105 continuously interacts with the interface module 106 so that, when the former is alerted by the later that user inputs are submitted, it determines whether such inputs represent changes that apply to the geometrical model, so-called geometry-related changes, or to the physics-related environment, so-called physics-related changes. For instance, when the interface module 106 detects that user inputs are submitted via the three-dimensional representation 202 of the geometrical model displayed in window 201, it alerts the update module 105 that, in turn, modifies the geometrical model defined by the geometry module 101. In other words, numerical and graphical inputs are continuously monitored by the interface module 106 and processed in real-time by the update module 105 to update the simulation environment (geometry and/or physics). Thus, the update module 105, by allowing changes made to the simulation environment to be continuously monitored and processed by the system, provides automatic updating mechanisms which contribute to increase efficiency of the system.

Another role of the update module 105 is to retrieve data from remote data sources, for instance medical measurement devices or a medical imaging devices. As previously stated, the system 100 may indeed be part of a computerized therapeutic environment, in which it may be connected to medical devices from which, by means of functionalities provided by the update module 105, it may retrieve geometrical or physics-related data.

In such situations, the update module 105 is configured to interact with interfaces of those devices and it updates the geometrical model defined by the geometry module 101 or the physics-related environment defined by the physics module 102 in accordance with data that it retrieves from those data sources. Thus, by allowing for instance in-vivo data to be directly retrieved and processed by the system, the update module 105 also contributes to increase efficiency of the system 100, especially when it is used in a such computerized R&D or medical environment.

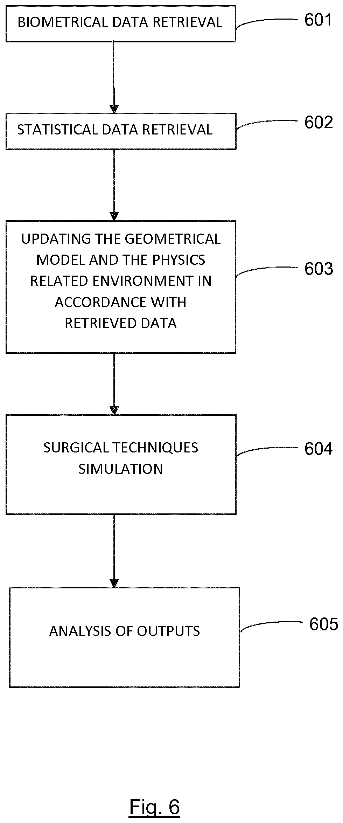

For the sake of providing a more complete picture of the use of the system in a therapeutic environment, Figure 6 depicts an example about how the system may be used as a tool in a process of corrective eye surgery, in particular within a surgical planning process. In a such process, the update module 105 first retrieves, in a step 601, biometrical data characterizing a patient's eye from a remote source such as a medical imaging device or any other appropriate system or instrument that may be used for gathering in-situ measurements of such physiological data. Then, in a step 602, the update module 105 retrieves also statistical data characterizing several physics-related parameters, for instance statistical data characterizing a refractive index parameter in relation to the patient's age. Such statistical data is stored in a dedicated database that is part of the system 100. Such data is built on the basis of studies performed on representative samples of patients, accumulated imaging and classified in accordance with several criteria such as age, ethnicity, gender, etc. Advantageously, the statistical data may be correlated to the patient to be treated by extracting from it mean values in relation to the patient's profile. Accordingly, following the step of data retrieval, step 602 may include an additional step of sorting the statistical data extracted from the database in relation to one or more characteristics that are specific to the patient to be treated, for instance the patient's age or the patient's gender. Then, in a step 603, as explained above, the update module 105 is configured to update the geometrical model hosted by the geometry module 101 and the physics-related environment managed by physics module 102 in accordance with the data that has been retrieved. Then, in a step 603, the physics module 102 may perform the computational procedure that has been described in relation to figure 3 to determine the best refractive index parameter. Alternatively, or cumulatively, step 603 includes a visual accommodation computation as described in relation to figure 5. Following this step, the geometrical model and the physics-related environment accurately reflect the anatomy of the patient's eye in terms of geometry and in terms of optical and mechanical properties before surgical treatment. Then, in a step 604, the surgeon provides inputs to the system which are meant to define surgical techniques that may be implemented. Those inputs are submitted via the interface module 106 and numerical or graphical outputs are computed by the simulation engine 103 via implementation of the computational steps that have been described in relation to figure 5. In a step 605, the outputs provided by the outputs providing module 104 are then analyzed in order to determine if, for instance, surgical techniques provide the desired result in terms of visual accommodation. This analysis may be performed either manually by the surgeon himself or automatically via an additional analysis module of the system 100 (not represented) that is configured to determine if a result fulfills certain predefined criteria, Then, through numerous iterations of steps 604 and 605, with respect to different surgical techniques that may be implemented, the surgeon is able to determine, for each patient, the best approach that must be followed in order to reach the desired result. In other terms, the system of the invention allows treatment's solutions to be investigated iteratively and results to be accurately forecasted and optimized without needing invasive steps to be performed.

In contrast to most system for simulating visual accommodation, which often focus only on the role of the crystalline the lens, the system 100 of the invention addresses with a great level of details numerous physiological entities that are present in a real eye. Moreover, as explained above, the geometry of the eye may be controlled from measured data or whatever data that is entered, which allows the system 100 of the invention to be able to grasp most of the possible geometrical combinations that may be found in real eyes. The system 100 of the invention is thus more efficient when used, for instance, in a R&D or therapeutic environment because, in a such situation, simulation results are useful only if they are able to reflect in an accurate manner the physiological reality.

## Claims

1. A computer system (100) for simulating visual accommodation,
**characterized in that**
said system comprises one or more processors and one or more computer-readable storage media encoded with instructions that, when executed by at least one of the processors, cause the computer system at least to:
- retrieve a pre-defined geometrical model, said pre-defined geometrical model delineating volumetric boundaries of a set of physiological entities of an eye, said set including at least a crystalline lens, a zonula, a ciliary muscle, a sclera and a cornea;
- determine if at least one geometry-related change is set and, if so, alter said pre-defined geometrical model using said geometry-related change to set an updated geometrical model;
- retrieve a physics-related environment, said physics related environment including at least one physics-related parameter assigned per physiological entity of said set of physiological entities, said physics-related parameter characterizing a mechanical property or an optical property;
- determine if at least one physics-related change is set and, if so, alter said physics-related environment using said physics-related change to set an updated physics-related environment; and
- combine said pre-defined geometrical model, or said updated geometrical model, and said physics-related environment, or said updated physics-related environment, to establish a simulation model.

2. The computer system of claim 1, wherein said instructions, when executed by at least one of the processors, further cause the computer system to display a graphical user interface in which at least a three-dimensional representation of an eye is presented, said graphical interface being configured to contain at least one input area for submitting said geometry-related change and/or said physics-related change.

3. The computer system of claim 2, wherein said instructions, when executed by at least one of the processors, further cause the computer system to detect when said geometry-related change results from an action performed in relation to said three-dimensional representation.

4. The computer system of one of the preceding claims, wherein said instructions, when executed by at least one of the processors, further cause the computer system to retrieve said geometry-related change and/or said physics-related change from a remote source.

5. The computer system of one of the preceding claims, wherein said instructions, when executed by at least one of the processors, further cause the computer system to compute, using said simulation model, at least one simulation result of visual accommodation, said simulation result containing data that, in relation to one physiological entity of said set of physiological entities, relates to a force, a mechanical stress, a deformation or a displacement, and/or, in relation to an optical domain, relates to an optical change, and/or relates to an accommodation amplitude value of said eye.

6. The computer system of claim 5, wherein said simulation result is computed on the basis of a first value of tension applied on a posterior part of said zonula and a second value of contraction of said ciliary muscle.

7. The computer system of one of the preceding claims, wherein said physics-related parameter or said physics-related change relates to a contraction level of said ciliary muscle and/or an elongation level of a posterior part of said zonula.

8. The computer system of one of the preceding claims, wherein said physics-related parameter or said physics-related change relates to a contraction and/or elongation level of a part of said ciliary muscle.

9. The computer system of one of the preceding claims, wherein said geometry-related change relates to said crystalline lens' shape and/or said sclera's shape.

10. The computer system of one of the preceding claims, wherein said geometry-related change relates to said cornea shape and/or said zonula's length.

11. The computer system of one of the preceding claims, wherein said geometry-related change relates to at least one position where said ciliary muscle, or said crystalline lens, is connected to said zonula.

12. The computer system of one of the preceding claims, wherein said crystalline lens includes at least a nucleus, a lens cortex, a lens capsule and a lens epithelium.

13. The computer system of one of the preceding claims, wherein said set of physiological entities includes a vitreous body and an aqueous humor.

14. The computer system of claim 13, wherein said physics-related parameter or said physics-related change relates to said aqueous humor's viscosity, said aqueous humor elasticity and/or a viscoelastic property of said vitreous body.

15. The computer system of one of the preceding claims, wherein said physics-related parameter or said physics-related change applies to only a part of said physiological entity to which it is assigned.
